Europäisches Patentamt

**European Patent Office**

**Office européen des brevets**

(19)

(11) Publication number: **0 196 921**
A2

(12) # EUROPEAN PATENT APPLICATION

(21) Application number: **86302432.9**

(22) Date of filing: **02.04.86**

(51) Int. Cl.⁴: **C 12 N 15/00**
C 12 N 9/64, C 12 N 1/20
C 12 N 5/00, C 07 K 5/00
C 07 K 7/00
//C12N9/99

(30) Priority: **03.04.85 US 719414**

(43) Date of publication of application:
**08.10.86 Bulletin 86/41**

(84) Designated Contracting States:
**AT BE CH DE FR GB IT LI LU NL SE**

(71) Applicant: **BIOTECHNOLOGY RESEARCH ASSOCIATES, J.V.**
**2450 Bayshore Frontage Road**
**Mountain View California 94043(US)**

(72) Inventor: **Fritz, Lawrence C.**
**20740 4th Street No. 2**
**Saratoga California 95070(US)**

(74) Representative: **Harrison, David Christopher et al,**
**MEWBURN ELLIS & CO 2/3 Cursitor Street**
**London EC4A 1BQ(GB)**

(54) **Recombinant human renin.**

(57) Expression vectors and methods for effecting the production of recombinant renin, prorenin, and preprorenin are disclosed. Sufficient quantities are thus obtained to generate pharmaceuticals useful in the treatment of disorders mediated by excessive renin levels such as hypertension. Methods to purify renin and prorenin from cell supernatants and to increase mature renin secretion are also disclosed.

FIG. 2

-1-

## RECOMBINANT HUMAN RENIN

### Technical Field

This invention relates to the production of practical quantities of scarce valuable proteins using recombinant means. In particular, the invention relates to the production of human renin in amounts sufficient to permit its use as a reagent in diagnosis and in drug design.

### Background Art

Renin is a critical protein in the "angiotensin system" which controls vasoconstriction/dilation. Accordingly, proposed therapies for disorders which are evidenced by improper levels of tension in the vascular system, notably hypertension, have focused on the control of this system.

The overall features of the angiotensin system for blood pressure regulation are understood. The vasoactive agent is an octapeptide, angiotensin II, which acts directly to constrict vascular smooth muscle, and which also induces the release of aldosterone from the adrenal cortex. The levels of angiotensin II are determined by the rate of inactivation by angiotensinases and the rate of formation in a two step process from angiotensinogen. The rate limiting step in the conversion of angiotensinogen to angiotensin II is the step catalyzed by renin--i.e., the conversion of angiotensinogen to angiotensin I. (The second step, conversion of angiotensin I to angiotensin II is mediated by "converting enzyme".) Renin is secreted in vivo by the juxtaglomerular cells of the kidney. It is synthesized as a prorenin precursor, which is then processed to give the active renin form.

The various features of the foregoing angiotensin system offer a number of opportunities for targeting of therapeutic drugs. Various approaches have, in fact, been tried. A large family of drugs, ß adrenergic inhibitors (ß-blockers) have been used extensively, and are known to act, at least in part, by inhibiting renin secretion from the juxtaglomerular cells of the kidney. Converting enzyme inhibitors have been used; examples of converting enzyme inhibitors include captopril and enalapril; substances which interfere with angiotensin II action directly include saralasin, which is a competitive inhibitor of angiotensin II binding. In vitro and in vivo studies have been conducted relating to direct renin inhibitors, such as antirenin antibodies, and angiotensin I analogs. However, these have not resulted in satisfactory therapeutically useful materials.

None of the therapies presently available presents a satisfactory solution to the problem of hypertension. Those substances which are actually in use as drugs are of unpredictable efficacy, and often have unwanted side effects due to a multiplicity of biological activities in addition to that intended. Some cannot be administered orally. This is particularly true of the renin inhibitors, some of which have been shown to be active in lowering blood pressure in both animals and humans when administered intravenously or intramuscularly (Haber, E., Hypertension and the Angiotensin System: Therapeutic

<u>Approaches</u> (1984) Raven Press 133-145; Gagnol, J. P., et al, <u>Abstracts International Society of Hypertension</u> (1984) <u>10</u>:376).

Renin itself is a secreted protein which has a precursor "prorenin" form. The protein is, therefore, translated initially as "preprorenin". Human kidney preprorenin contains, from the N-terminal end, a 20 amino acid residue signal sequence which is lost upon secretion, a "pro" segment of 46 amino acid residues which is cleaved <u>in vivo</u> and is susceptible to <u>in vitro</u> cleavage by trypsin, and the mature protein containing 340 amino acids (Imai, T., et al, <u>Proc Natl Acad Sci</u> <u>(USA)</u> (1983) <u>80</u>:7405). Prorenin or renin is presumably secreted as a glycoprotein, as evidenced by its affinity for concanavalin A-sepharose. Mature human kidney renin has a molecular weight of 37,200 daltons without its sugar residues and shows a molecular weight of approximately 40,000 in its glycosylated form (Yokosawa, H., et al, <u>J Biol Chem</u> (1980) <u>255</u>:3498-3502).

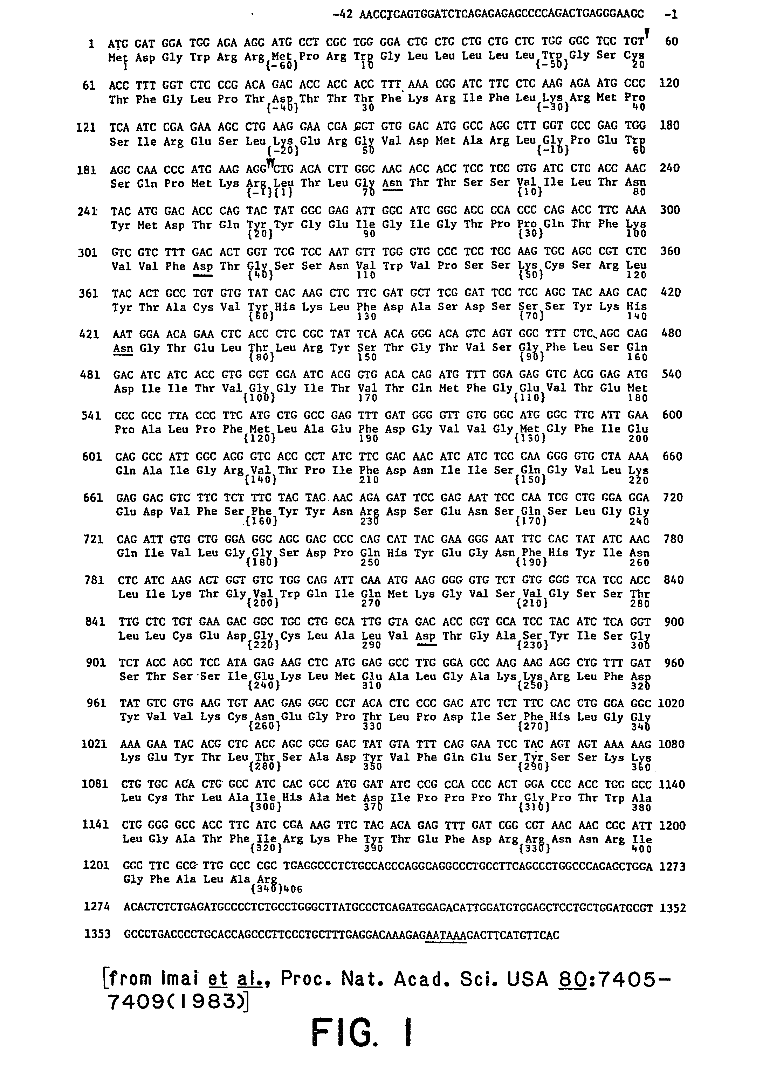

cDNA for human kidney renin has been prepared and the complete nucleotide sequence has been determined (Imai, T., et al, (supra)). This sequence and the deduced protein sequence are shown in Figure 1. However, none of renin, prorenin, and preprorenin has been prepared using recombinant techniques, and the human renin obtained by purification has been limited in quantity to that which can be isolated from plasma or kidney. A protein having renin activity prepared from the mouse submaxillary gland has been crystallized and preliminary X-ray diffraction data obtained; three dimensional models of the human protein have been proposed by analogy (Carlson, W., et al, <u>Abstracts</u> <u>International Society Hypertension</u> (1984) <u>10</u>:153; Murakami, K., ibid (1984) <u>10</u>:154). Insufficient human

-4-

renin material has been available, however, to permit experimental determination of its three dimensional structure.

The art at present lacks a means to provide practicable quantities of human kidney renin or its precursors sufficient to provide substrate for determination of three dimensional structure, and design of appropriate drugs based on this conformation. The general approach of basing design of specific interacting ligands on the spatial configuration of the target material has been successfully used with other proteins, including the design of ligands specifically binding to the 2,3-diphosphoglycerate site in human hemoglobin (Beddell, C. R., et al, Brit J Pharmacol (1976) 57:201-209; Beddell, C. R., ibid (1979) 65:535-543; anti-cancer pharmaceuticals which bind specifically to dihydrofolate reductase (Hansch, C., et al, J Med Chem (1982) 25:777-784; thyroid hormone analogs which bind to prealbumin (Blaney, J. M., et al, J Med Chem (1982) 25:785-790; and elastase inhibitors (Hughes, D. L., et al, J Mol Biol (1982) 162:645-658).

Relatively poor inhibitors consisting of the renin pro sequence have also been made for mouse renin and human renin by Evin, et al, Proc Natl Acad Sci USA (1984) 81:48-52, and by Boger, et al, Nature (1983) 303:81-84. The location of the pepsinogen "pro" sequence at the active site of pepsin as disclosed by James, M., et al, Nature (1986) 319:33-38, however indicates that this relatively poor performance could be overcome if the pure crystalline protein were available in sufficient quantity to permit adequate study of the active site.

In addition, it is desirable to obtain large quantities of renin for use as standards in diagnostic assays for renin levels in the blood stream or other

-5-

fluids.  At present, these immunological assays are unstandardized by appropriate control materials.  Also, purified human renin is needed as a reagent in screening tests for potential renin inhibitors which screens will then be free of the interfering reactions exhibited by the impure preparations currently available.

It would also be desirable to obtain recombinant renin as a secreted mature protein.  The secretion of mature insulin from pituitary cells transformed with a vector encoding proinsulin has been obtained by treating these cells with a cyclic AMP analog as a secretagogue (Moore, H-P.H., et al, Cell (1983) 35:531-538).  The behavior of the recombinant insulin therefore apparently utilizes the secretion mechanism employed by these cells in producing ACTH and endorphin (Mains, R.E., et al, J Biol Chem (1981) 89:21-28).

In summary, there is at present no readily available source of purified human renin protein, and no source of the recombinant form of this protein, despite the need for such supplies in providing therapy and diagnosis of hypertensive or other vascular system diseases.

Disclosure of the Invention

The invention provides renin in sufficient quantity to supply the needs for this protein as currently realized.  Sufficient amounts are made available through recombinant techniques to permit adequate drug design and to supply quantities as diagnostic standards.  In addition, the availability of recombinant materials and methods to produce this substance provides the opportunity for modification of

-6-

the protein sequences to optimize the structure of the enzyme for whatever its intended purpose.

Accordingly, in one aspect, the invention relates to recombinantly produced renin and its precursors either in glycosylated or unglycosylated form. In other aspects, the invention relates to expression vectors suitable for producing these proteins, to hosts transformed with DNA sequences encoding them, to methods of recombinant renin, prorenin, and preprorenin production, and to therapeutic materials designed to complement the conformation of the recombinantly produced material. In another aspect, the invention relates to a method to effect secretion of mature renin for cultured mammalian cells. In still other aspects the invention relates to purification procedures for renin and prorenin, especially as recombinantly produced.

Brief Description of the Drawings

Figure 1 shows the complete nucleotide sequence of preprorenin encoding DNA, along with the deduced amino acid sequence.

Figure 2 shows the construction of an expression vector which encodes preprorenin and which results in secretion of prorenin from mammalian cell cultures.

Figures 3a and 3b show the construction of bacterial expression vectors for renin-related proteins.

Figure 4 shows an SDS-polyacrylamide gel of proteins immunoprecipitated from supernatant of CHO cells transformed with renin-encoding plasmids and grown in the presence of $^{35}$S-methionine.

Figure 5 shows SDS-polyacrylamide gels of proteins labeled with $^{35}$S-methionine extracted from E. coli transformed with renin-encoding vectors.

Figure 6 shows SDS-polyacrylamide gels of proteins labeled with $^{35}$S-methionine from the medium of AtT20 cells immunoprecipitated with anti-renin.

## Modes of Carrying Out the Invention

### A. Definitions

As used herein "renin" refers to a protein having an amino acid sequence substantially similar to that shown in Figure 1 as the mature protein--i.e., the 340 amino acid sequence beginning at position 66. It is, of course, understood that renin, like all proteins, may be present in neutral or salt forms, depending upon the pH of its surroundings or of the solutions from which it is obtained. Both neutral and salt forms are included in the definition. In addition, like many proteins, renin may exist in conjugation with other molecules, most prominently carbohydrate residues--i.e., in glycosylated or unglycosylated form. Other "side chain modifications" may also occur, such as, for example, conjugation with phosphate, oxidation of, for example, sulfhydryl residues, esterification of hydroxyls, and so forth. These modifications are also included as long as "renin activity", as defined by the ability to catalyze the conversion of angiotensinogen to angiotensin I is not destroyed. Finally, it is known that the sequence itself may be altered in minor ways, for example, addition, deletion, or substitution of one or more amino acid residues. Such altered forms are also included as long as they retain renin activity. In particular, it is understood that as is the case for many proteins, only a portion of the mature amino acid sequence may be required for activity.

"Prorenin" and "preprorenin" are defined correspondingly with reference to Figure 1. (The corresponding numbers referred to in the figure are those below the amino acid residue, and absent brackets.) Thus, the preprorenin is represented by the entire amino acid sequence shown and prorenin that from residue 21 to residue 406. The mature sequence is that from residue 66 to residue 406. It is assumed that the 20 amino acid leader presequence from residue 1 through 20 is cleaved as the protein is translocated across the rough endoplasmic reticulum; the "pro" sequence from residue 21 to 66 is cleavable in vitro using a proteolytic enzyme of the proper specificity, such as trypsin. Enzymatically inactive renin, presumably prorenin, is, in fact, found in the plasma (Sealey, J. E., et al, Encocrine Rev (1980) 1:365-391) and in the kidney itself (Chang, J. J., et al, Hypertension (1981) 3:509-515). It appears that some, but not all, of the prorenin found in human plasma is of kidney origin (Derkx, F. H. M., et al, Hypertension (1983) 5:244-256).

Renin activity can be assayed by incubation with human angiotensinogen and measuring the angiotensinI (AI) generated using a standard immunoassay.

"Purified" or "pure" refers to material which is free from substances which normally accompany it as found in its native state. Thus "pure" renin, for example, refers to renin which does not contain materials normally associated with its in situ environment in human plasma or kidney. Of course, "pure" renin may include materials in specific association with it, such as its glycoside residues.

-9-

"Operably linked" refers to a juxtaposition wherein the components are configured so as to perform their usual function. Thus, control sequences or promoters operably linked to a coding sequence are capable of effecting the expression of the coding sequence.

"Control sequence" refers to a DNA sequence or sequences which are capable, when properly ligated to a desired coding sequence, of effecting its expression in hosts compatible with such sequences. Such control sequences include promoters in both procaryotic and eucaryotic hosts, and, in procaryotic organisms, also include ribosome binding site sequences, and, in eucaryotes, termination signals. Additional factors necessary or helpful in effecting expression may also be identified. As used herein, "control sequences" simply refers to whatever DNA sequence may be required to effect expression in the particular host used.

"Expression system" refers to a DNA sequence which contains DNA encoding a desired product operably linked to any control sequences that may be necessary to effect expression of the coding sequence.

"Cells" or "cell cultures" or "recombinant host cells" or "host cells" are often used interchangeably as will be clear from the context. These terms include the immediate subject cell, and, of course, the progeny thereof. It is understood that not all progeny are exactly identical to the parental cell, due to chance mutations or differences in environment. However, such altered progeny are included in these terms, so long as the progeny retain the characteristics relevant to those conferred on the originally transformed cell.

-10-

## B. General Description

In general, the invention is directed to the production of purified forms of renin (or prorenin) in practical amounts and free from impurities which accompany such protein in native surroundings. Recombinant techniques offer an ideal approach to reach this goal. The DNA sequences encoding the desired renin or prorenin product may be ligated into suitable vectors and expressed in either procaryotic or eucaryotic hosts, thus providing an almost unlimited source of these proteins. The presequence may also be used advantageously, especially in eucaryotes, to effect secretion of the desired product. Secretion is, of course, desirable, as it permits an easy and straightforward first purification step consisting of simply removing the culture medium from the cells. The number of proteins secreted by most recombinant host cells is limited, so that the level of initial contamination will be significantly lower for secreted proteins.

Secreted protein from mammalian cell pools typified by CHO cells should be incubated with or without trypsin for to assay for renin activity, since CHO cells secrete renin almost completely in the inactive pro form and trypsin converts prorenin to renin. Renin activity in supernatants from CHO cells can be increased 25-fold by treatment of the secreted protein with trypsin prior to assay. A variety of host and control systems is available; the preferred control system employs the metallothionein promoter. Culturing these cells containing preprorenin sequences under control of the metallothionein promoter in the presence of $2-7.5 \times 10^{-5}$ M $ZnSO_4$ increases renin secretion by 2-3 fold. Inclusion of $10^{-6}$ M dexamethasone to the

-11-

culture also results in an additional 3-5 fold increase in renin production. Secretion of the prorenin and renin products into serum-free medium using this system is also feasible in the presence of an induction activator such as iron.

When prepared in mammalian cells which utilize the regulated pathway for secretion (as opposed to the constituitive pathway - for a review see Kelly,____, et al, Science (198 ) 230:25-32), the secretion of mature protein, rather than prorenin, is stimulated using a secretagogue. For example, in the illustration below, it has been found that such a secretagogue, a cyclic AMP analog, is capable of effecting secretion of renin in mature form from a pituitary cell line transformed with an expression system for preprorenin. It is believed that the mature recombinant renin, like other mature hormones that are ordinarily secreted from these cells, is stored in secretory granules which respond to the secretagogue by disgorging their contents into the culture supernatant.

The invention includes a method to effect secretion of mature renin from suitably transformed cells originating in tissues which employ the regulated secretion pathway, such as those of the endocrine and exocrine glands, for example, hypothalamus, parathyroid, heart atrium, and so forth, or neurons, basophils, and platelets. To effect secretion, after production of the recombinant protein has been maintained for a suitable length of time, the cells are treated with a secretagogue to release the mature form. Secretagogues are generally known in the art and include cyclic AMP analogs, calcium ionophores, and norepinephrine. These compounds are supplied in an amount effective to obtain release, dependent on the secretagogue used.

-12-

Any of the cDNA sequence, the genomic sequence, or hybrids thereof may be expressed depending on the nature of the recombinant host and the control systems used. In the illustration below, cDNA is prepared from kidney cell mRNA and probed with the appropriate genomic sequences. cDNA has also been prepared by Murakami (see Imai (supra)). However, mammalian cell hosts, for example, are capable of expressing intron-containing genomic sequences, and producing properly spliced mRNA.

Purification methods for obtaining the mature or pro- form of renin from recombinant cultures are also disclosed. For renin, the procedure comprises hydrophobic interaction chromatography (HIC), pepstatin chromatography, and anion exchange. For prorenin, HIC is inappropriate since conversion to the mature protein occurs. Therefore, prorenin is prepared by sequential application of pepstatin chromatography, size exclusion, and anion exchange.

For renin, a culture supernatant is treated to remove cell debris, and then applied to a hydrophobic column, such as Phenyl 5PW in salt solution, and then eluted with a decreasing salt gradient in pH 8 buffer. A number of suitable adsorbants is commercially available, for example, Synchropak Propyl (Synchrom, Linden, IN) and Phenyl Superose (Pharmacia). The fractions containing prorenin (or renin) are recovered, treated with trypsin (for example attached to Sepharose beads) and then applied to a pepstatin column. Pepstatin is an inhibitor which is capable of binding renin and activated prorenin effectively; this is essentially an affinity step. A variety of supports can be used to employ the pepstatin. The renin is eluted with dilute acid, and then subjected to anion exchange chromatography at about pH 7; it is obtained in pure form in the flow-through volume. Any convenient anion exchange resin may be used, such as DEAE or other ion exchanger, many of which are readily commercially available. Exemplary are Beckman SAX Ultrasil and Synchrom AX300.

For prorenin, the clarified culture supernatant is applied to the pepstatin column, which is eluted with acid, as above, and the prorenin-containing fractions are subjected to size exclusion HPLC using, for example, TSK 4000 SW or other comparable sizing gel capable of excluding both high and low molecular weight contaminants, such as Toyo Soda μ-Spherogel TSK or Beckman Biosil 250, and then anion exchange to obtain the prorenin in the flow-through volume as above.

The desirability of obtaining pure recombinant human renin and prorenin has several facets. First, milligram amounts of the material are obtainable using this procedure. Milligram amounts are capable of crystallization to permit three dimensional studies using X-ray diffraction and computer analysis. This permits deductions concerning the shape of the molecule, thus defining proper shapes for substances useable as inhibitors of the enzyme activity normally exhibited by renin. Inhibitors have already been designed for "converting enzyme", the catalyst for the subsequent conversion of angiotensin I into angiotensin II. Generally, these antagonists have been "dipeptides" whose interactions with converting enzyme are stabilized by modification of the "residues" participating in the peptide bond so as to enhance the ability of the "dipeptide" to interact specifically with converting enzyme. Thus the peptide bond joins specifically chosen carboxylic acids and amines (not necessarily amino acids). These "dipeptides" are configured in a three

-14-

dimensional array so as to complement the contours of the intended target, converting enzyme. A similar lock and key spatial arrangement will result from molecules designed complementary to the surface contours of the crystallized renin of the invention. It is understood that "surface" includes convolutions which may face inward, and specifically includes the active site. Furthermore, "complementary" is understood to mean that in addition to spatial conformations which "fit", interactions between the protein and the molecule which matches its surface contours are attractive and positive. These interactions may be hydrogen bonding, ionic, or hydrophobic affinity.

Accordingly, the invention contemplates analogous stabilized peptide antagonists (2-15 amino acids) to renin which are characterized by three dimensional contours complementary to the three dimensional contours on the surface of recombinant renin (or prorenin). By peptide in this context is meant that the antagonist contains carboxylic acid amide bonds corresponding to one less than the number of residues. The carboxylic acid and amine participants need not be α-amino acids. A particularly preferred source for the peptides is the amino acid sequence of the "pro" portion of prorenin.

Second, human renin has an exquisitely specific enzymatic activity, which, however, differs significantly from the activity of nonprimate renins. Human renin cleaves the Leu-Val bond in human angiotensinogen, and the Leu-Leu bond in nonprimate angiotensinogens. No other known substrates exist. Nonprimate renins, for example, mouse submaxilliary gland renin, do not cleave human angiotensinogen. Accordingly, human renin has a

-15-

unique specificity which differs from that of nonprimate renins.

Third, even without the assistance of a three dimensional structure determination, purified renin of the invention is of significance as a reagent in screening renin inhibitors in vitro as an ad hoc approach to evaluation. Impure renin preparations currently available yield confusing data due to the impact of the impurities on the test results. For example, contaminants which turn out to be themselves inhibitors, activators, or substrates for renin will interfere with the evaluation. Thus, a substantial improvement in current screening techniques for renin inhibitors would be effected by the availability of the purified human renin protein.

In addition, because the availability of materials for recombinant production of a protein permits sequence modification, for example, by site-specific mutagenesis, modification of characteristics of the product protein, such as its substrate specificity is possible. Therefore the recombinant vectors of the invention provide the starting materials to obtain a series of activating proteases for a spectrum of processing conversions.

Finally, the recombinant renin of the invention is also useful in providing a specific and sensitive diagnostic assay for human renin in biological samples. There is, at present, no commercially available direct immunoassay for renin in the bloodstream, although immunoassays have been used in research. Knowledge of renin or prorenin levels is of great importance in determining appropriate therapies (Laragh, J. H., Hypertension and the Angiotensin System: Therapeutic Approaches (1984) Raven Press pp 46-69). The clinical assays now used are based on renin activity, i.e., are enzyme assays, and subject to considerable error. In order to provide to the clinician the direct immunoassay approach, now available only for research, it will be necessary to provide suitable standards of purified human renin (Menard, J., et al, Abstracts International Society Hypertension (1984) 10:152; Ojihara, T., et al, (ibid) (1984) 10:47; Galen, F. X., et al, J Clin Endocrinol Metab (1979) 48:1041-1043). The availability of purified recombinant prorenin and renin will provide this material for standardization and calibration, both of the direct immunoassay and of currently used renin activity assays. The level of prorenin or renin in the bloodstream thus can be used both as a preliminary diagnosis to assess the disorder, and also as a monitoring system to track the effectiveness of therapeutic treatment.

C.   Standard Methods

Most of the techniques which are used to transform cells, construct vectors, extract messenger RNA, prepare cDNA libraries, and the like are widely practiced in the art, and most practitioners are familiar with the standard resource materials which describe specific conditions and procedures. However, for convenience, the following paragraphs may serve as a guideline.

C.1.   Hosts and Control Sequences

Both procaryotic and eucaryotic systems may be used to express the renin encoding sequences; procaryotic hosts are, of course, the most convenient for cloning procedures. Procaryotes most frequently are represented by various strains of E. coli; however, other microbial strains may also be used. Plasmid vectors which contain replication sites and control sequences derived from a species compatible with the host are used; for example, E. coli is typically transformed using derivatives of pBR322, a plasmid derived from an E. coli species by Bolivar, et al, Gene (1977) 2:95. pBR322 contains genes for ampicillin and tetracycline resistance, and thus provides additional markers which can be either retained or destroyed in constructing the desired vector. Commonly used procaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang, et al, Nature (1977) 198:1056 and the tryptophan (trp) promoter system (Goeddel, et al Nucleic Acids Res (1980) 8:4057 and the lambda derived $P_L$ promoter and N-gene ribosome binding site (Shimatake, et al, Nature (1981) 292:128).

In addition to bacteria, eucaryotic microbes, such as yeast, may also be used as hosts. Laboratory strains of Saccharomyces cerevisiae, Baker's yeast, are most used although a number of other strains are commonly available. Vectors employing, for example, the 2 μ origin of replication of Broach, J. R., Meth Enz (1983) 101:307, or other yeast compatible origins of replications (see, for example, Stinchcomb, et al, Nature (1979) 282:39, Tschempe, et al, Gene (1980) 10:157 and Clarke, L, et al, Meth Enz (1983) 101:300) may be used. Control sequences for yeast vectors include promoters for the synthesis of glycolytic enzymes (Hess, et al, J Adv Enzyme Reg (1968) 7:149; Holland, et al, Biochemistry (1978) 17:4900).

Additional promoters known in the art include the promoter for 3-phosphoglycerate kinase (Hitzeman, et al, J Biol Chem (1980) 255:2073), and those for other glycolytic enzymes. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization. It is also believed terminator sequences are desirable at the 3' end of the coding sequences. Such terminators are found in the 3' untranslated region following the coding sequences in yeast-derived genes.

It is also, of course, possible to express genes encoding polypeptides in eucaryotic host cell cultures derived from multicellular organisms. See, for example, Axel, et al, 4,399,216. These systems have the additional advantage of the ability to splice out introns and thus can be used directly to express genomic fragments. Useful host cell lines include VERO and HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papiloma virus, or avian sarcoma viruses. The controllable promoter, hMTII (Karin, M., et al, Nature (1982) 299:797-802) may also be used. General aspects of mammalian cell host system transformations have been described by Axel (supra). It now appears, also that "enhancer" regions are important in optimizing expression; these are, generally, sequences found upstream or downstream of the promoter region in non-coding DNA regions. Origins of replication may be obtained, if needed, from viral sources. However, integration into the chromosome is a common mechanism for DNA replication in eucaryotes.

### C.2. Transformations

Depending on the host cell used, transformation is done using standard techniques appropriate to such cells. The calcium treatment employing calcium chloride, as described by Cohen, S. N., Proc Natl Acad Sci (USA) (1972) 69:2110, or the $RbCl_2$ method described in Maniatis, et al, Molecular Cloning: A Laboratory Manual (1982) Cold Spring Harbor Press, p. 254 may be used for procaryotes or other cells which contain substantial cell wall barriers. For mammalian cells without such cell walls, the calcium phosphate precipitation method of Graham and van der Eb, Virology (1978) 52:546, optionally as modified by Wigler, M., et al, Cell (1979) 16:777-785 may be used. Transformations into yeast may be carried out according to the method of Van Solingen, P., et al, J Bact (1977) 130:946 or of Hsiao, C. L., et al, Proc Natl Acad Sci (USA) (1979) 76:3829.

### C.3. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

The DNA sequences which form the vectors are available from a number of sources. Backbone vectors

-20-

and control systems are generally found on available "host" vectors which are used for the bulk of the sequences in construction. Typical sequences have been set forth in ¶C.1 above. For the pertinent coding sequence, initial construction may be, and usually is, a matter of retrieving the appropriate sequences from cDNA or genomic DNA libraries. However, once the sequence is disclosed it is possible to synthesize the entire gene sequence in vitro starting from the individual nucleoside derivatives. The entire gene sequence for genes of sizeable length, e.g., 500-1000 bp may be prepared by synthesizing individual overlapping complementary oligonucleotides and filling in single stranded nonoverlapping portions using DNA polymerase in the presence of the deoxyribonucleotide triphosphates. This approach has been used successfully in the construction of several genes of known sequence. See, for example, Edge, M. D., Nature (1981) 292:756; Nambair, K. P., et al, Science (1984) 223:1299; Jay, Ernest, J Biol Chem (1984) 259:6311.

Synthetic oligonucleotides are prepared by the method of Efimov, V. A., et al (Nucleic Acids Res (1982) 6875-6894), and can be prepared using commercially available automated oligonucleotide synthesizers. Kinasing of single strands prior to annealing or for labeling is achieved using an excess, e.g., approximately 10 units of polynucleotide kinase to 1 nmole substrate in the presence of 50 mM Tris, pH 7.6, 10 mM $MgCl_2$, 5 mM dithiothreitol, 1-2 mM ATP, 1.7 pmoles γ32P-ATP (2.9 mCi/mmole), 0.1 mM spermidine, 0.1 mM EDTA.

Once the components of the desired vectors are thus available, they can be excised and ligated using standard restriction and ligation procedures.

-21-

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM $MgCl_2$, 6 mM DTT and 5-10 µM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow,

-22-

the mixture is extracted with phenol/chloroform and ethanol precipitated. Treatment under appropriate conditions with S1 nuclease or Bal-31 results in hydrolysis of any single-stranded portion.

Ligations are performed in 15-50 μl volumes under the following standard conditions and temperatures: for example, 20 mM Tris-Cl pH 7.5, 10 mM MgCl$_2$, 10 mM DTT, 33 μg/ml BSA, 10 mM-50 mM NaCl, and either 40 μM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 μg/ml total DNA concentrations (5-100 nM total end concentration). Intermolecular blunt end ligations are performed at 1 μM total ends concentration.

In vector construction employing "vector fragments", the vector fragment is commonly treated with bacterial alkaline phosphatase (BAP) or calf intestinal alkaline phosphatase (CIP) in order to remove the 5' phosphate and prevent religation of the vector. Digestions are conducted at pH 8 in approximately 150 mM Tris, in the presence of Na$^+$ and Mg$^{+2}$ using about 1 unit of BAP or CIP per μg of vector at 60° for about one hour. In order to recover the nucleic acid fragments, the preparation is extracted with phenol/chloroform and ethanol precipitated. Alternatively, religation can be prevented in vectors which have been double digested by additional restriction enzyme digestion and separation of the unwanted fragments.

For portions of vectors derived from cDNA or genomic DNA which require sequence modifications, site specific primer directed mutagenesis may be used. This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered.

### C.4. Verification of Construction

In the constructions set forth below, correct ligations for plasmid construction are confirmed by first transforming E. coli strain MC1061 obtained from Dr. M. Casadaban (Casadaban, M., et al, J Mol Biol (1980) 138:179-207) or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, optionally following chloramphenicol amplification (Clewell, D. B., *J Bacteriol* (1972) *110*:667). The isolated DNA is analyzed by restriction and/or sequenced by the dideoxy method of Sanger, F., et al, *Proc Natl Acad Sci (USA)* (1977) *74*:5463 as further described by Messing, et al, *Nucleic Acids Res* (1981) *9*:309, or by the method of Maxam, et al, *Methods in Enzymology* (1980) *65*:499.

### C.5. Hosts Exemplified

Host strains used in cloning and expression herein are as follows:

For cloning and sequencing, and for expression of construction under control of most bacterial promoters, *E. coli* strain MC1061 was used.

The cells used for mammalian expression are Chinese hamster ovary (CHO) cells or a human pituitary cell line designated AtT-20.

### D. Illustrative Procedure

The following examples are intended to illustrate but not to limit the invention. The DNA encoding preprorenin and its processed products is obtained by probing a cDNA library constructed from kidney cell mRNA. However, it would not be necessary to repeat this procedure, as the sequence is now known, and cells harboring one vector containing the complete coding sequence of human prorenin preceeded by a modified human signal sequence are deposited at the American Type Culture Collection. CBI-2B5, harboring pPP14 was deposited with ATCC 26 March 1985 and has accession no. CRL 8758. Methods to obtain entire gene sequences, once the desired sequence is known are now available. See, e.g., Edge, M. D., et al, *Nature* (1981) 292:756; Nambiar, K. P., et al, Science (1984) 223:1299; or Jay, E., et al, J Biol Chem (1984) 259:6311.

### D.1. Preparation of the Preprorenin Coding Sequence

A cDNA library was prepared from oligo dT primed polyA$^+$ RNA, purified from the kidney of a human accident victim. The cDNA library was constructed in the bacteriophage vector λgt10 and probed with appropriate fragments of Charon-4 human renin genomic clones which had been obtained from a human genomic library by probing with mouse submaxillary gland renin cDNA fragments. The library was prepared by standard procedures to obtain double-stranded cDNA from the mRNA, blunt-ending the cDNA with DNA polymeraseI (Klenow), adding commercially available EcoRI linkers, cleaving with EcoRI, and ligating the fragments into EcoRI-digested λgt10 vectors. Two positively responding cDNA clones, together, when sequenced using the dideoxy method of Sanger (supra), were shown to contain the entire renin encoding sequence and 3' untranslated region except for a missing 7 base pairs at the 5' end of the signal sequence. The two clones include 1211 bp of coding region, followed by the entire 3' 198 bp untranslated region followed, in turn, by a polyA tail. The assignment of the sequence to the appropriate preprorenin codons and reading frame was made by comparison to the published Imai, et al (supra) sequence. Two cloned fragments result because of the unique EcoRI site at position 762 as shown in Figure 1.

(It has been, further, shown, by probing the human genomic library that the genomic structure is complex, and contains 10 coding exons. Genomic clones were not used in the constructions below.)

-26-

The cDNA sequences encoding the preprorenin protein were transferred into pUC9 as the two EcoRI inserts to obtain the cloned vectors pHR1 and pHR2. pHR1 contains the 5' portion and pHR2 contains the 3' portion. pHR1 and pHR2 were prepared by excising the cDNA from λgt10 using EcoRI and ligating each of the resulting fragments into EcoRI-cleaved pUC9.

### D.2. Construction of a Mammalian Expression Vector for Preprorenin

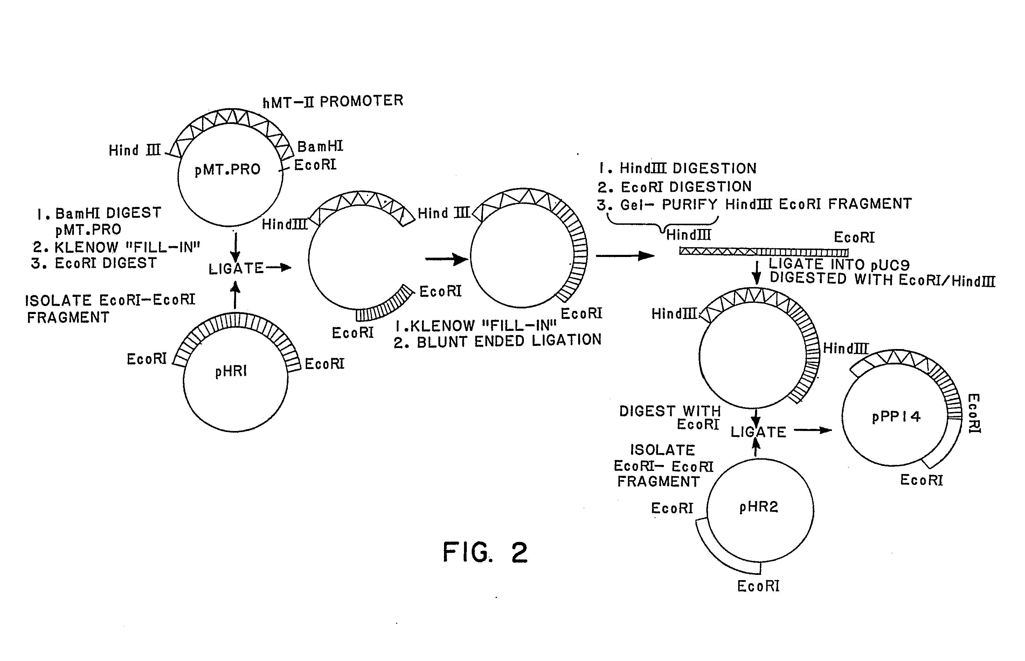

The construction of pPP14, an expression vector for preprorenin compatible with mammalian hosts and having the coding sequences under the control of the human metallothionein-II (hMT-II) promoter is shown in Figure 2. To construct pPP14, pMT.PRO was used as a host expression vector. pMT.PRO is similar to pHS1, described in U.S. serial no. 701,296, filed 13 February 1985, and to pMT401, described in U.S. serial nos. 616,488 and 622,639, filed 1 June 1984 and 20 June 1984, respectively. All of the foregoing U.S. patent applications are assigned to entities controlled by the same assignee as the herein application, and are incorporated herein by reference.

Both of the pHS1 and pMT401 plasmids contain 840 bp of the hMT-II sequence from p84H (Karin, M., et al, Nature (1982) 299:297-802) which spans from the HindIII site at position -765 of the hMT-II gene to base +70. pMT.PRO contains a similar hMT-II fragment which, however, further includes the ATG start codon when ligated into pUC8. All three vectors are obtained by ligation of the appropriate hMT-II sequences into pUC8 (Vieira, J., et al, Gene (1982) 19:259-268). To construct pMT.PRO, the hMT-II sequence was excised from pMTII-BPV (Karin, M., et al, Proc Natl Acad Sci (USA) (1983) 80:4040-4044) as a HindIII/HindIII fragment, which was then digested with BamHI to obtain the promoter and 5' transcript portion. The HindIII/BamHI fragment containing the hMT-II sequences was then inserted into HindIII/BamHI-digested pUC8 to obtain pMT.PRO.

To construct pPP14, pMT.PRO was digested with BamHI, filled in using DNA polymerase (Klenow) in the presence of the four dNTPs, and then digested with EcoRI. EcoRI cuts immediately downstream from the unique BamHI site which follows the ATG start codon. The 5' portion of the preprorenin gene was excised from pHR1 by EcoRI digestion, and ligated under sticky-end conditions to the EcoRI tail of the opened pMT.PRO vector. Approximately 50% of the inserts will be in the correct orientation. The ligation mixture was then treated with DNA polymerase I (Klenow) in the presence of the four dNTPs, to blunt the remaining unligated EcoRI site. Finally, the vector was recircularized by ligation, and the mixture transformed into E. coli MC1061 to Amp$^R$. Colonies containing the correct construction, with the 5'-portion of the preprorenin coding sequences in reading frame with the ATG provided by the hMT-II promoter region and 5' transcript, were picked and cultured. Construction of the intermediate plasmid was confirmed by restriction analysis and sequencing.

The resulting intermediate plasmid seemed unable to accept the EcoRI-excised 3' portion obtained from pHR-2 in the correct orientation. This problem was overcome by replacing the backbone vector of the intermediate, which had been derived from pUC8, with the corresponding sequences from pUC9. To do this, the intermediate vector was treated with HindIII and EcoRI, and the fragment containing the hMT-II promoter/preprorenin 5' portion inserted into HindIII/EcoRI digested pUC9. The resulting modified intermediate was then digested with EcoRI and ligated with the EcoRI/EcoRI fragment isolated from pHR2 to generate pPP14, which contains the entire preprorenin coding sequence.

Since the junction region between the ATG start codon of the pMT.PRO and the 5' terminal portion of the preprorenin encoding sequences were joined by blunt-end ligation after repair of the BamHI and EcoRI cleaved sites, respectively, the sequence in the junction region

is 5'-CC<u>ATG</u>GATCAATTCCGATGG, thus placing the underlined ATG start codon shown in reading frame with the remainder of the coding sequence. The overlining indicates the junction of the filled in BamHI and EcoRI sites. Thus the coding sequence for the N-terminus of the preprotein of the signal sequence is Met-asp-gln-phe-arg-trp, which contains two additional amino acids in comparison to the native corresponding N-terminal sequence, which is Met-asp-gly-trp.

The resulting vector, pPP14, was then used as described below for expression of preprorenin in host mammalian cells, including Chinese hamster ovary (CHO) and AtT-20 cells.

"Chinese Hamster Ovary" (CHO) cells include the standard cell line ATCC CCL-61, and its relatives isolated from the same source tissue, as well as derivatives thereof. Derivatives are mutants of the line which may differ genotypically or phenotypically from the original line, but which are obtained therefrom by intentional or inadvertent mutation.

Similarly AtT-20 cells include ATCC cell line CCL-89 and its relatives and derivatives as described above.

The pPP14 construction has also been modified by inserting a 1120 bp HindIII/HindIII fragment from SV40 (bases 5171-1046) into the HindIII site upstream of the promoter. The inserted fragment contains the SV40 enhancer region.

### D.3. Construction of Bacterial Expression Vectors for Renin and Prorenin

Two bacterial expression vectors were prepared, one for mature renin (designated pLF5) and the other for prorenin (designated pAA6). In both cases, the host plasmid was pKT52, which contains the "trc" promoter followed by an ATG. The construction of pKT52 is described in U.S. serial nos. 616,488 and 622,639 (supra). Briefly, the "trc" promoter contains the upstream portions of the trp promoter and the downstream, operator-containing, regions of the lac promoter and was originally prepared from two readily available plasmids containing these promoters. To construct the trc promoter as a BamHI/HindIII cassette, an intermediate plasmid pKK10-0 was prepared containing the hybrid promoter.

To prepare pKK10-0, pEA300 (Amman, E., et al, Gene (1983) 25:167-178) was digested with PvuII and ClaI, filled in using dCTP only in the presence of DNA polymerase (Klenow), followed by digestion with mungbean nuclease, and the large vector fragment isolated. This vector fragment contains the upstream portions of the trp promoter. The fragment was ligated with a 55 bp blunt-ended HpaII/PvuII digest excised from pGL101 (Lauer, G., et al, J Mol Appl Genet (1981) 1:139-147), which was prepared by digesting pGL101 with PvuII and HpaII followed by repair in the presence of dGTP and labeled dCTP. This fragment contains the lac operator region. The ligation product of these two blunt-end fragments was pKK10-0.

A BamHI site was inserted into pKK10-0 upstream of the trp/lac (trc) promoter/operator by digestion with EcoRI, filling in with Klenow, and insertion of the BamHI linker 5'-CCGGATCCGG. The resulting plasmid, pKK10-1 was digested with PvuII, and ligated to the NcoI linker, 5'-ACCATGGT, digested with NcoI, filled in, and then ligated to a double-stranded linker containing PstI and HindIII sites provided as two complementary oligonucleotides, 5'-GCTGCAGCCAAGCTTGG-3' and its complement. The ligation mixture was transformed into E. coli to Amp$^R$. The isolated plasmid DNA was digested with BamHI and HindIII, and the small BamHI/HindIII fragment obtained on electrophoresis contains the trc promoter.

To complete pKT52, the BamHI/HindIII fragment containing the trc promoter was ligated into the large fragment obtained from BamHI/HindIII digestion of pKK10-2 (Brosius, J., Gene (1984) 27:161-172) which contains the Amp$^R$ gene and the origin of replication. The resulting plasmid, pKK233-1 was digested to completion with PvuI and then partially with BglI and ligated with the 360 bp PvuI/BglI fragment containing the corresponding portion of the ampicillin resistance gene but lacking a PstI site from pUC8. The ligation mixture was used to transform E. coli and transformants were screened for the presence of only one PstI site next to the trc promoter. The correct construction, pKK233-2, was digested with EcoRI and PvuII, filled in

-31-

with dATP and dTTP, and religated to obtain the correct construction pKT52.

pKT52 contains the desired trc promoter, a downstream ATG start codon, and downstream NcoI, PstI and HindIII sites.

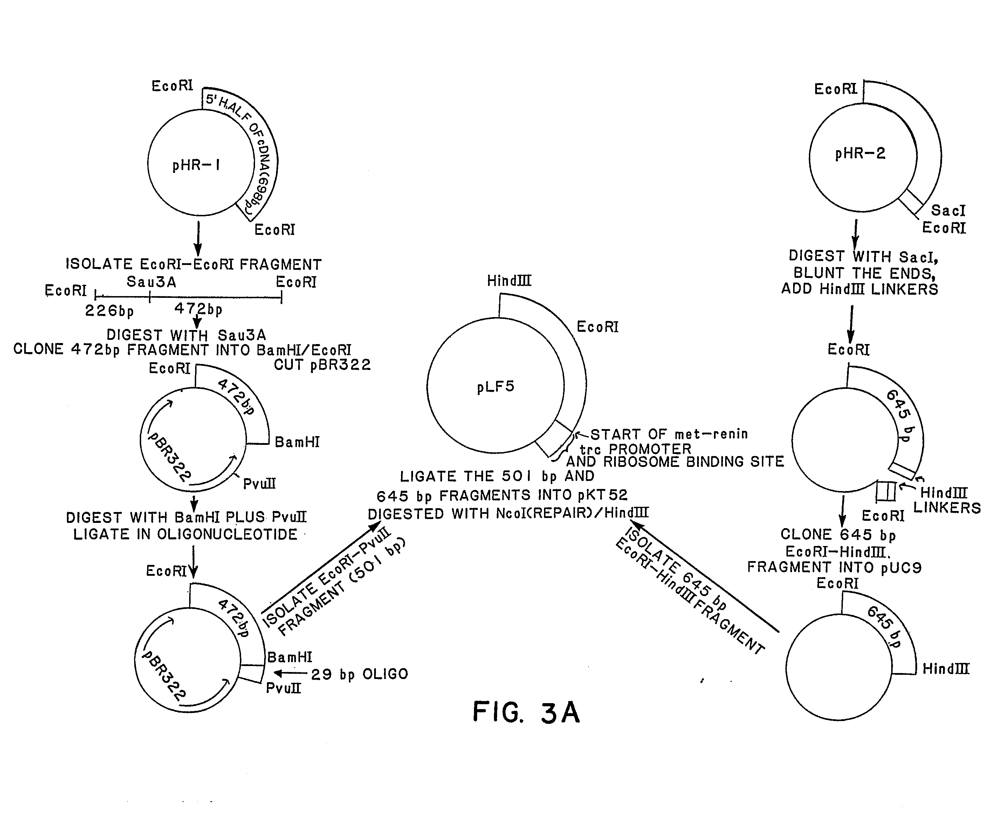

For pLF5 (shown in Figure 3a), the renin coding sequences were prepared as follows: the 5' portion of renin cDNA was excised from pHR1 by EcoRI digestion and isolation of the 698 bp fragment. The fragment was digested with Sau3AI to liberate the 472 bp downstream fragment which begins nine codons into the mature renin coding sequence. This fragment was then ligated into BamHI/EcoRI-digested pBR322. The resulting intermediate plasmid was digested with BamHI/PvuII and the paired synthetic complementary oligonucleotides

5'-CTGACCCTTGGCAACACCACCTCTTCTGT

GACTGGGAACCGTTGTGGTGGAGAAGACACTAG-5',

which provide the missing codons, was ligated into the digested plasmid. The PvuII site proximal to the upstream terminus is recreated by this procedure. The resulting intermediate plasmid was digested with EcoRI and PvuII to yield a 501 bp fragment containing the 5' coding sequences for mature renin.

The 3' terminal sequences were obtained as a 645 bp fragment by EcoRI/HindIII digestion of a pHR2 derivative in which pHR2 had been modified by cleaving with SacI, blunt ending with T4 polymerase, adding HindIII linkers, and cleaving with HindIII. The EcoRI/HindIII digest was cloned into pUC9 which had also been digested with EcoRI and HindIII.

The two portions were then ligated into an NcoI(repair)/HindIII digest of pKT52, where pKT52 then provides the blunt ATG downstream of the trc promoter. (See Figure 3a.) The resulting expression plasmid, pLF5, contains the entire renin coding sequence in reading frame with the ATG start codon under the control of the trc promoter.

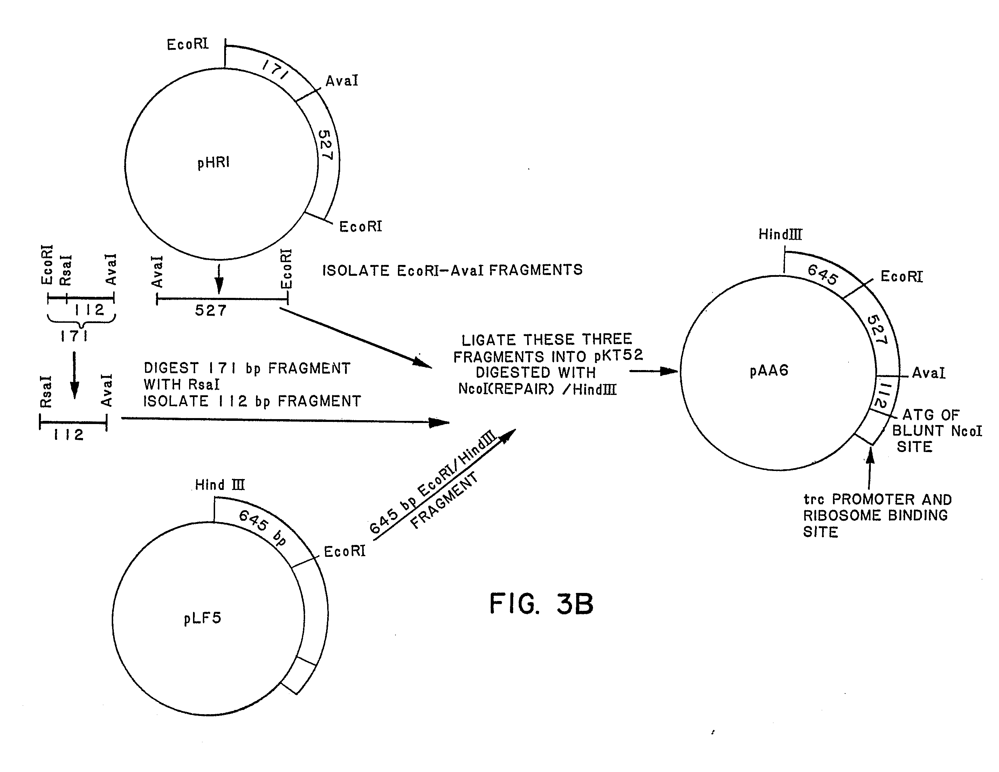

To obtain pAA6 (Figure 3b), a procaryotic expression vector for prorenin, pHR1 was digested with AvaI and EcoRI to obtain a 171 bp EcoRI/AvaI fragment and a 527 bp AvaI/EcoRI fragment. After separation on a 5% acrylamide gel and electroelution, the 171 bp fragment was further restricted with RsaI which cleaves at the start site of prorenin yielding a 112 bp fragment.

The above-described 527 bp and 112 bp fragments were ligated, along with a 645 bp fragment generated by EcoRI/HindIII digestion of pLF5 to provide the downstream coding sequences, into the above-described NcoI (repair)/HindIII digest of pKT52. The resulting vector, pAA6 contains the prorenin sequences in frame with the ATG start codon of pKT52 and under the control of the trc promoter. (See Figure 3b.)

### D.4. Expression of Preprorenin Sequences in Mammalian Cells

#### Plate Assays - CHO Cells

The mammalian expression vector pPP14 was used to transform CHO cells grown in 50% Dulbecco's modified Eagles medium/50% Coon's F12 medium supplemented with 10% fetal calf serum. Twenty μg of plasmid (pPP14 DNA) together with 2 μg of the marker plasmid pSV2:Neo were applied to the cells in 100 mm dishes using the standard calcium phosphate precipitation technique (supra) and a 15% glycerol shock 3 hr after DNA application. Twenty-four hr after transfection, the cells were treated with 0.4 mg/ml of G418 antibiotic and G418 resistant colonies were expanded and analyzed for production of the desired protein.

The G418-resistant colonies were induced with $1-5 \times 10^{-5}$ M $ZnSO_4$ to obtain protein production. The medium of the induced cells was supplemented with $^{35}$S-methionine, to label the synthesized protein, the culture medium was removed and a portion reacted with antibody prepared either against native renin (anti-renin antibodies) or against a synthetic peptide representing the C-terminal 12 amino acids of the propeptide sequence (anti-"pro" antibodies). The immunoprecipitates were subjected to SDS-gel electrophoresis, and the bands detected by autoradiography. Both anti-renin and anti-"pro" antibodies yielded bands of appropriate molecular weight corresponding to prorenin.

Another portion of the labeled medium was treated first with 50 μg/ml trypsin in Tris buffer, pH8, for 60 min at 25°C. The trypsinized media were then treated with either anti-renin or anti-"pro" antibodies and the immunoprecipitates subjected to SDS-gel electrophoresis, as above. The anti-renin antibodies now immunoprecipitated a protein shown to have the molecular weight of mature renin. Anti-pro antiserum did not immunoprecipitate this mature form.

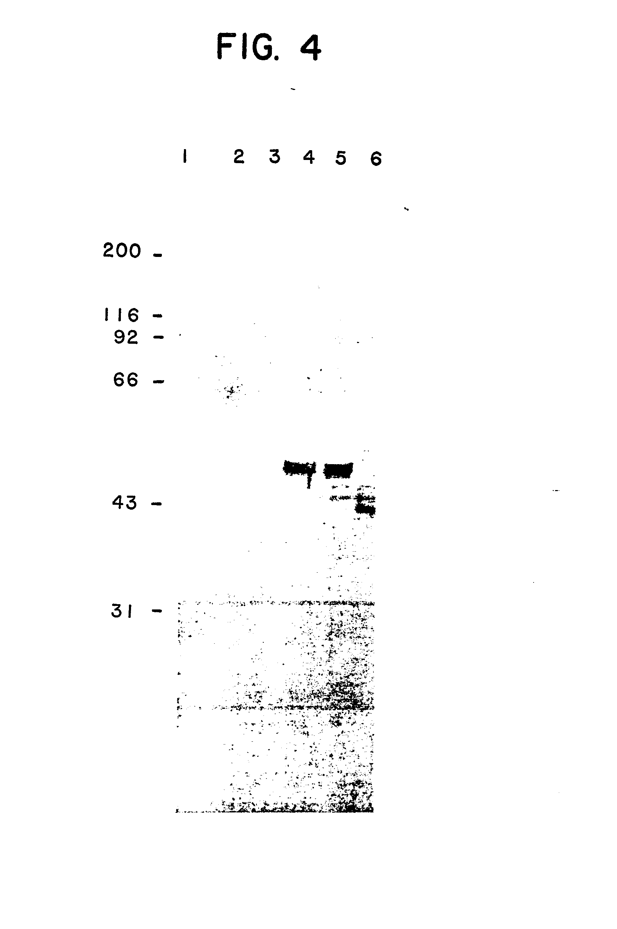

Figure 4 shows these results. Lanes 1-3 represent immunoprecipitates of medium from cells transformed with control plasmid; lanes 4-6 are immunoprecipitates of media from cells transformed with pPP14. Lanes 1 and 4, and 2 and 5 represent immunoprecipitation with anti-pro antibodies and antirenin antibodies respectively. (Anti-human renin antibodies described in Dzau, V. J., et al, <u>Clin Exp Hypertension</u> (1983) A5(7-8):1207-1220 were supplied by Dr. Victor Dzau, Harvard Medical School.) Lanes 3 and 6 are gels of immunoprecipitates obtained from trypsin-treated media using anti-renin antibodies.

In general pools of CHO cells selected for G418 resistance after transformation with pPP14 produce renin after induction with $5 \times 10^{-5}$ zinc sulfate and $10^{-6}$ M dexamethasone at levels of 0.02-0.2 µg/ml. The renin so produced was shown to be glycosylated by comparison of SDS gels run on $^{35}$S methionine-labeled secreted renin with and without treatment with endo F.

By appropriate selection, much higher levels of renin production can be achieved. In one approach, cells from the transformant pool were plated out in the presence of $5 \times 10^{-5}$ M zinc sulfate in serum-free medium, and colonies which grew under these conditions were picked, expanded in serum-containing medium, and then assayed for renin production under standard induction conditions as described above. The highest renin-producing clones obtained after this process produced an average of 0.8 µg/ml, and one clone of this class, designated CBI-2B5 (supra) was deposited at ATCC with accession no. CRL 8758.

The culture medium of the pPP14-transformed CHO cells designated CBI-2B5 grown in the absence of radioactive label was also directly tested for renin activity. Samples which were pretreated with trypsin under the conditions described above were used in the assay; media from cells transfected with plasmid not containing renin sequences were used as controls. In this assay, human angiotensinogen is supplied as a substrate which, in the presence of renin, is converted to angiotensin I. The angiotensin I product is measured using a competitive immunoassay kit marketed by Genentech-Travenol with $^{125}$I-labeled angiotensin I as the tracer. Media from CBI-2B5 cells treated with trypsin showed renin activity in this assay at levels of 30-50 x $10^3$ ng angiotensin/hr/ml culture supernatant when assayed at 25% maximal substrate concentration. Trypsin-treated media from cells transformed with control plasmid had no detectable activity.

Another high producer cell line was obtained as follows: The cells from the pool were plated and replicated onto a polyester sheet. The replica sheet was then placed against a nitrocellulose filter for binding of the secreted cell protein. The filter was then reacted with with antiprorenin antiserum followed by treating with $^{125}$I protein A. Clones which appeared to have the highest levels of renin secretion according to this assay were picked, expanded in serum-containing medium, and reassayed for activity under standard conditions. Clones were obtained producing 1-4 µg/ml of renin, and only one such clone, arbitrarily chosen, CBI-AA2, was selected for study under production conditions, as described below.

### AtT-20 Transformants

AtT-20 cells were grown as described for CHO cells above in preparation for transformation, except that a 15% $CO_2$ atmosphere rather than a 5% $CO_2$ atmosphere was used. Transformation and selection for successful transformants by G418 resistance was as described for CHO cells except as noted below, and the selected cells were screened for renin production in the supernatant fluid using the plate assays described for the CHO cells producing renin above, except that only serum-containing media were used.

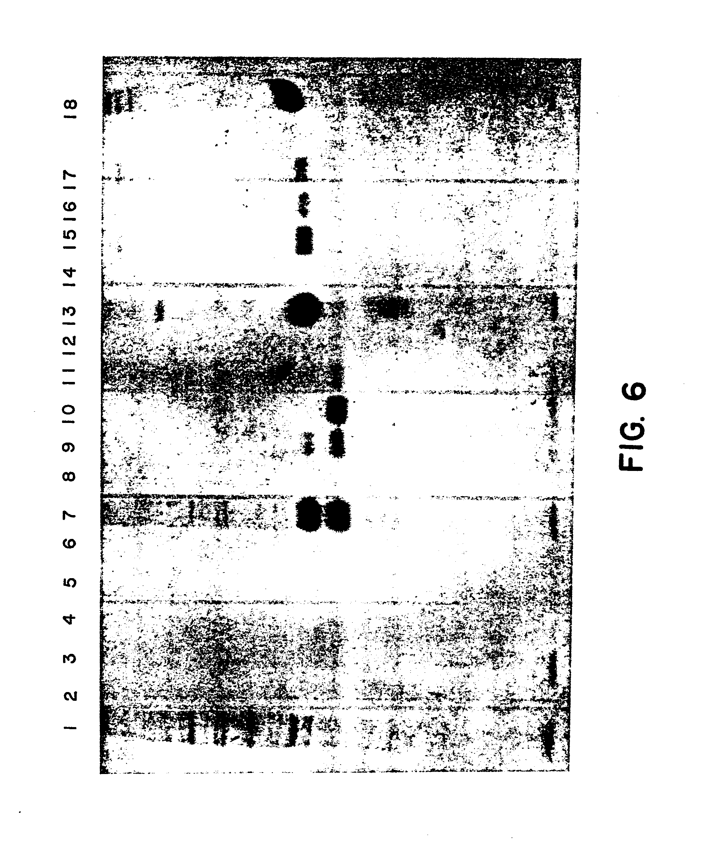

High producing lines secreted both renin and prorenin into the medium; the secretion of renin could

-36-

be dramatically increased by treating the cells with 8-bromo cAMP. A comparison of the secreted material precipitable with human renin antiserum is shown in Figure 6, described below.

The cells were grown as described above, seeded at $10^6$ cells per plate, and washed the next day with DME21 with 10% FCS. The cells were transfected with 125 µg pPP-14 and 25 µg pSV2NEO and selected in 250 µg/ml G418. After induction for expression as described above, and accumulation of recombinant product, the cultures were treated or not with 5 mM 8-bromo-cAMP, and after 3 hr, the supernatants were assayed for renin activity in the presence and absence of trypsin. (Activity without trypsin represents mature renin, activity in the presence of trypsin represents the sum of renin and prorenin.) The results are as follows:

|  | Renin Activity ng AI/ml/hr | |
|---|---|---|
|  | + trypsin | − trypsin |
| −8Br−cAMP | 15.3 | 6.8 |
| +8Br−cAMP | 45.6 | 38.8 |

Thus addition of the secretagogue results in secretion of approximately an additional 30 ng AI/ml/hr in renin activity.

Figure 6 shows an SDS gel of immunoprecipitated proteins from supernatants of $^{35}$S-methionine labeled cells as follows: untransfected AtT-20 cells, AtT-20 cells transfected with pPP-14, and CHO cells transformed with this plasmid. The cells were labeled for 15 hr with $^S35$-methionine after induction as described above. The medium was collected, and the supernatant

-37-

immunoprecipitated with a rabbit anti-human renin antiserum and protein A sepharose and subjected to 10% SDS-PAGE.

Lane 2 represents the immunoprecipitates from these initial supernatants from untransformed cells, lane 7 from transformed AtT-20 cells, and lane 13 from transformed CHO cells. As shown, the transfected AtT-20 cells secrete both prorenin and renin; transfected CHO cells secrete only the prorenin form.

Wells were washed with unlabeled medium, and incubated for 3 hr in fresh unlabeled medium. At the end of the 3 hr period, this medium was collected (lanes 3, 9 and 15). Identical wells were then treated for 3 hr with fresh medium containing either 5 mM 8-bromo cAMP (lanes 4, 10 and 16) or 0 mM 8-bromo cAMP (lanes 5, 11 and 17).

As shown in Figure 6, secretion of labelled renin or prorenin fell off as expected after removal of $^{35}$S-methionine (lanes 9 and 15). Continued incubation resulted in further diminuition (lanes 11 and 17) but treatment with 5 mM Br-cAMP resulted in secretion of labeled renin from AtT-20 cells (lane 10) but not of prerenin from CHO cells (lane 16).

AtT-20 cultures containing successful transformants are also subjected to selection for cells capable of being maintained under serum free conditions. The cultures are grown in 10% FCS to 1/2 confluence, and then switched to serum-free medium. The culture plates are fed with serum-free medium until most but not all cells have died, whereon medium containing 10% FCS is added to allow division of surviving cells. The surviving cells are passaged, replated, and after 1 day on 10% FCS are again switched to serum-free medium. Cells which survive 2 weeks are expanded by addition of medium containing 10% FCS, cloned at limiting dilution, and assayed for renin production after induction using zinc ion and iron as induction mediator as described above.

## Production Level Renin Cultures

One vial of stock cells containing ~1.0 x $10^7$ cells in one ml of freezing media is defrosted rapidly by agitation at 37°C. Cells are divided equally and plated into two 100 mm tissue culture dishes containing 10 ml each of complete media. (Complete media consists of 10% fetal calf serum in a 1:1 mixture of Coon's F-12/Dulbecco's modified Eagle media with 4.5 mg/ml glucose (DMEM-21). Antibiotic consisting of 50 units/ml penicillin/50 µg/ml streptomycin or 400 µg/ml G418 may be added as desired. Stock media older than two weeks is supplemented with 2 mM gluatmine. Defrosted cells are incubated at 37°C in a 5% $CO_2$ incubator until the cells have attached (~30 min). Media are aspirated and the cells are refed with fresh complete media.

Cells are allowed to grow to confluence and are passaged with trypsin by standard protocols for expansion. Cells are passaged at a dilution of >10 and <40 as needed. Stock cells expanded for long-term storage are frozen according to standard protocols.

850 $cm^2$ roller bottles are seeded with cells from one confluent T-150 flask (~4 x $10^7$ cells). Corning brand roller bottles are preferred. Cells are passaged with trypsin by standard protocols and seeded in 200 ml of complete media supplemented with 10 mM Hepes pH 7.2. Cells are grown in a 37° room on a roller rack rotating at ~6-7 min per revolution until confluent.

Serum-free inducing media (SFIM) for increased renin production by CBI-2B5 consists of the following:

10 mm Hepes, pH 7.2 (1 M stock Hepes is filtered through a 0.2 n filter to sterilize and is stored at 4°C), $3 \times 10^{-5}$ M $FeSO_4$ (1000 x stock $FeSO_4$ is prepared in $H_2O$, immediately sterile filtered, aliquoted into 1.5 ml aliquots in cell freezing vials, and stored at -80°C. $5 \times 10^{-5}$ $ZnSO_4$ (a 200 x stock is prepared in $H_2O$, sterile filtered, aliquoted, and stored at 4°C; $10^{-6}$ M dexamethasone (a 1000 x stock is prepared in 100% ethanol and stored at 4°C); Coon's F12/DMEM 21 (1:1); and glutamine supplement as needed, antibiotic as desired.

Cells are switched to serum-free media as follows:

Complete media are aspirated from the roller bottle, and 200 ml DMEM 21 added. The bottle is returned to the roller rack for 10 min before aspirating the media and refeeding with 100-200 ml SFIM. The cells are refed again when the cells have conditioned the media with secreted protein, ~100 mcg/ml total protein as determined by Bradford assay. (About two days per 100 ml for CBI-2B5 at the beginning of the roller run, but the time required for the cells to condition the media may decrease with time post switch.) The cells are refed by aspirating or pipetting off the media very gently (the cells become very loosely attached to the bottle with time), and adding back 100-200 ml SFIM.

The CHO transformant cells designated CBI-AA2 were prepared for roller bottle culture growth in 100 mm tissue culture dishes containing 10 ml complete media (10% FCS in F12/DMEM21 and containing 50 U/ml penicillin, 50 µg/ml streptomycin, or 400 µg/ml G-418. The cells are grown until attached (30 min), the media withdrawn, and cells refed with fresh complete media. The cells were allowed to grow to confluence, and passaged by trypsin using standard protocols for expansion. 850-cm square roller bottles were seeded with cells from one confluent flask (4 x $10^7$ cells) in 200 ml complete medium supplemented with 10 mM Hepes, pH 7.2. The cells were grown at 37°C with rotation until confluent.

To induce production of renin in serum-free medium, the complete media were aspirated from the roller bottles and the cells were washed with 200 ml DMEM21. The cells were then fed with approximately 100-200 ml serum-free induction medium (SFIM). SFIM consists of F12/DMEM21 (1:1), containing 1 mM Hepes, 3 x $10^{-5}$ M $FeSO_4$, 5 x $10^{-5}$ M $ZnSO_4$, and $10^{-6}$ M dexamethasone. The cells are refed with SFIM on a cycle determined by the amount of secreted protein -- i.e., when approximately 100 µg/ml total protein has been secreted, as determined by Bradford assay. In general this level of total protein production occurs in approximately 2 days. CBI-AA2 showed renin production levels of 1 µg/ml or approximately 0.5 µg/ml/day.

### Purification of Renin

Renin was purified from serum-free CHO cell supernatants by the sequential application of hydrophobic interaction chromatography, pepstatin affinity chromatography, and anion exchange chromatography.

### HIC

Specifically, 1.5 M NaCl was added to CHO supernatants, and dissolved by gentle swirling at room temperature. The solution was filtered through an 8

μm prefilter (Sartorius Sartopure capsule), and 0.45 μm filters (Sartorius cellulose nitrate), using a Sartorius ultrafiltration apparatus with peristaltic pump. The filtrate was stored at 4°C, until ready for hydrophobic interaction chromatography (HIC). Each HIC run utilized 4 liters of filtered supernatant, to which 0.02% sodium azide were added. The column employed was a Phenyl 5PW preparative HPLC column (7.5 x 2.1 cm, Toyo Soda Company, Japan), placed into a Pharmacia FPLC chromatography system, which includes two P-500 FPLC pumps controlled with the LCC-500 chromatography controller. The sample was pumped directly onto the column at a flow rate of 6 ml/min, after equilibration with 1.5 M NaCl, 50 mM Tris-Cl, pH 8.0 (Buffer A). The absorbance of the eluant was monitored at 280 nm with the Pharmacia UV-1 absorbance detector, and solvent switching of pump A accomplished with motor valve MV-8. After loading 1.5 liters of supernatant, and re-equilibration of the column to Buffer A, bound proteins were eluted with a decreasing salt gradient: a 15 min linear gradient to 95% Buffer B (50 mM Tris-Cl, pH 8.0), followed by a 15 min linear gradient to 100% B. Fractions containing prorenin (as judged by trypsin-activatable renin activity) were collected and another 1.5 liters of supernatant were loaded.

### Pepstatin Column

The collected fractions from HIC were trypsin activated from prorenin to renin by adding trypsin conjugated Sepharose 4B beads (coupling procedure was as outlined in Pharmacia literature, utilizing approximately 20 mg/ml trypsin from Boehringer Mannheim). After centrifugation to remove the beads, the renin solution was dialyzed against 20 mM NaOAc pH 5.7 (loading buffer), and any resulting precipitate was removed by a second centrifugation. This sample (500 ml, from two 4-liter HIC runs) was loaded (at about 1 ml/min) onto a 40 ml pepstatin column equilibrated with loading buffer (column prepared by the procedure of Murakami, K., et al, Biochem Biophys Res Comm (1975) 62:757-763, utilizing Aminohexyl Sepharose as the support). After washing the column with 1 M NaCl in loading buffer, followed by loading buffer alone, the renin was eluted with 0.1 N acetic acid, directly into tubes containing neutralizing amounts of 2 M Tris-Cl, pH 8.0.

Anion Exchange

Eluate from the pepstatin column was further purified by ion exchange HPLC, utilizing an anion cartridge in a Brownlee NewGuard HPLC guard holder as guard, and DEAE 5 PW (7.5 x 0.75 cm, Toyo Soda Company) as analytical column. The HPLC system consisted of two Beckman/Altex 110A pumps with 421 controller, Eldex pump monitor, and Kratos Spectroflow 757 variable wavelength absorbance detector, set at 280 nm and connected to a Kipp and Zonen BD 40 chart recorder. Sample (about 100 ml, from 4 pepstatin runs) was pumped onto the column at 1 ml/min, after equilibration with 100 mM Triethylammonium acetate, pH 7.0. Flow through material exhibiting absorbance at 280 nm was collected, and contained essentially pure active renin with specific activity 400-900 Goldblatt Units per mg.

Purification of Prorenin

Due to substantial activation of prorenin to renin during passage over the HIC column, CHO supernatants were utilized without the HIC step for

-43-

purification of prorenin. The purification procedure utilizes pepstatin affinity, size exclusion, and ion exchange chromatography. Inactive renin is unable to bind to renin inhibitors, so the supernatants are activated by acid prior to loading onto pepstatin. One M glycine, pH 3.3, is added dropwise over 18 hr. at 4°C, to a stirred solution of supernatant until the solution is pH 3.3. This activated prorenin is loaded onto a pepstatin column previously equilibrated with 20 mM NaOAc, pH 5.7. After sequential washes with starting buffer, buffer plus 1 M NaCl, then buffer alone, the prorenin is eluted with 0.1 M acetic acid. The collected fractions containing prorenin are dryed under vacuum, and resuspended in a small volume of 100 mM Triethylammonium acetate, pH 7.0. As the fractions contain both high and low molecular weight contaminants, the next two steps will involve HPLC chromatography that has been shown to remove these contaminants from previous prorenin purifications: first, size exclusion chromatography on TSK 4000 SW column (Toyo Soda Company), in the above buffer, followed by addition of 1 M Tris-Cl pH 7.0 (to make a final concentration of 0.1 M Tris) and DEAE chromatography as described above for active renin. Flow-through fractions from this column should contain pure prorenin.

D.5.  Expression of Renin and Prorenin in Bacterial Cells

For bacterial expression, either pLF5 or pAA6 was used to transform E. coli MC1061 cells to $Amp^R$ and the transformed cells were grown on M9 medium containing 1 mM IPTG for 3-5 hours to an OD of 0.2-0.5. (IPTG is a standard inducer for control sequences regulated by lac operator.) The cells were then pulse-labeled with

$^{35}$S-methionine, harvested, lysed by sonication or treatment with 5% trichloroacetic acid, and the cell extracts assayed for the desired proteins by SDS-gel electrophoresis.

The extracts were treated with either anti-renin or anti-"pro" antibodies, and the immunoprecipitates subjected to SDS-gel electrophoresis. Cells transformed with pAA6 yielded immunoprecipitates with anti-"pro" antibodies which migrated on gels to positions corresponding to the correct molecular weight for prorenin. Cells transformed with pLF5 did not. Neither the extracts from pLF5 nor pAA6 yielded protein which immunoprecipitated with anti-renin antibodies. It is assumed this failure to precipitate is due to the conformation dependence of the anti-renin antibody that was used.

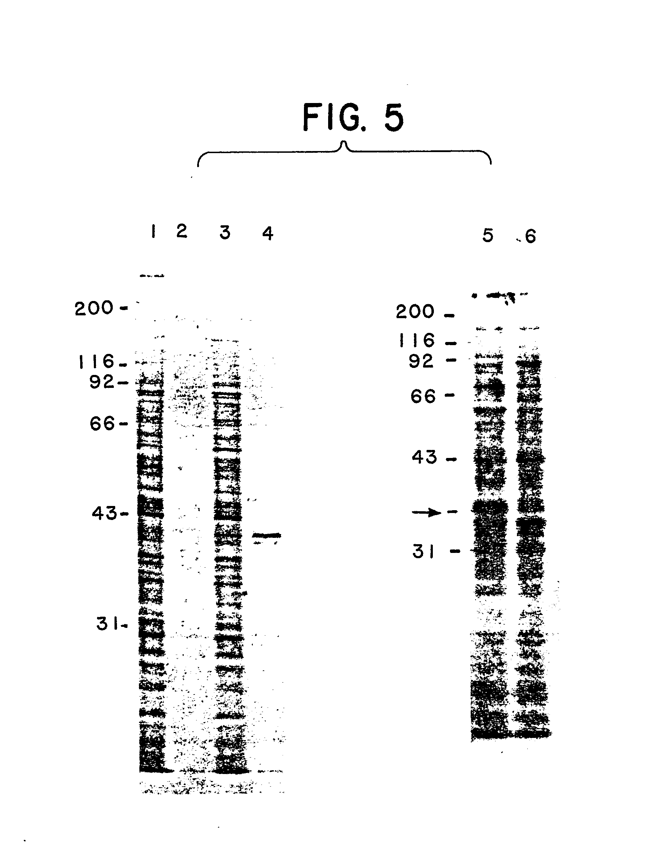

The results above are shown, generally, in figure 5. Lanes 1, 3, 5 and 6 represent total 5% TCA precipitated proteins from E. coli transformed with, respectively, pKT52, pAA6, pLF5 and pKT52. The presence of a band at approximately 42 kD molecular weight in lane 3 and of approximately 37 kD molecular weight in lane 5, which bands are absent in lanes 1 and 6, is clearly seen. Lanes 2 and 4 are gels run on immunoprecipitates with anti-pro antibodies from extracts of cells harboring pKT52 and pAA6 respectively. The immunoprecipitated protein from the pAA6 transformed organism migrates corresponding to the same molecular weight as the putative prorenin band in the total extract.

Protein corresponding to the 37 kD band referred to above was purified from bacteria containing pLF5 by differential centrifugation and preparative gel electrophoresis, eluted, and the resulting recovered

-45-

protein subjected to N-terminal sequencing. The N-terminal sequence of the protein thus obtained was Met-leu-thr-leu-gly-asn-thr-thr, which corresponds to the correct N-terminal sequence for Met-renin.

## Claims

1. An expression system which comprises the DNA sequence encoding human renin, prorenin, or preprorenin operably linked to a control sequence compatible with a recombinant host cell and capable of effecting the expression of said DNA sequence in said recombinant host cells.

2. The system of claim 1 wherein the control sequence is compatible with a mammalian host.

3. The system of claim 1 wherein the control sequence is compatible with a bacterial host.

4. Recombinant host cells transformed with a recombinant DNA sequence encoding renin, prorenin, or preprorenin.

5. The cells of claim 4 wherein the recombinant DNA sequence further includes control sequences operably linked to the DNA encoding renin, prorenin or preprorenin.

6. The cells of claim 4 which are mammalian cells.

7. The cells of claim 4 which are bacterial cells.

8. A method of preparing human renin or prorenin which comprises culturing the cells of claim 4 and recovering the renin or prorenin from the culture.

-47-

9. A peptide (2-15 amino acid residues) having a three dimensional surface contour which complements the three dimensional surface contour of a portion of the surface of crystallized recombinant renin, wherein said peptide is an inhibitor of renin activity.

10. A composition of matter which comprises recombinantly produced human renin or prorenin free from impurities.

11. A method to produce secreted, cultured mature recombinant renin which comprises treating mammalian cells which natively employ a regulated secretory pathway, which have been modified to contain an expression system for preprorenin, and which have been induced to express the preprorenin gene, with a secretagogue.

12. A method to purify renin from cell culture supernatant which method comprises hydrophobic interaction chromatography (HIC), pepstatin chromatography, and anion exchange.

13. A method to purify prorenin from cell culture supernatant which method comprises pepstatin chromatography, size exclusion, and anion exchange.

CLAIMS:

1.      A method of preparing human renin or prorenin which comprises culturing recombinant host cells transformed with a recombinant DNA sequence encoding renin, prorenin, or preprorenin and recovering the renin or prorenin from the culture.

2.      A method according to claim 1 wherein the recombinant DNA sequence further includes control sequences operably linked to the DNA encoding renin, prorenin or preprorenin.

3.      A method according to claim 1 or claim 2 wherein the cells are mammalian cells.

4.      A method according to claim 1 or claim 2 wherein the cells are bacterial cells.

5.      A method according to any one of the preceding claims wherein the human renin or prorenin is produced free from impurities.

6.      A method of producing secreted, cultured mature recombinant renin which comprises treating mammalian cells which natively employ a regulated secretory pathway, which have been modified to contain an expression system for preprorenin, and which have been induced to express the preprorenin gene, with a secretagogue.

7.      A method of purifying renin from cell culture supernatant which method comprises using hydrophobic interaction chromatography (HIC), pepstatin chromatography, and anion exchange.

8.      A method of purifying prorenin from cell culture supernatant which method comprises using pepstatin chromatography, size exclusion, and anion exchange.

9.     A peptide (2-15 amino acid residues) having a three dimensional surface contour which complements the three dimensional surface contour of a portion of the surface of crystallized recombinant renin, wherein said peptide is an inhibitor of renin activity.

```
          -42 AACCTCAGTGGATCTCAGAGAGAGCCCCAGACTGAGGGAAGC   -1

  1 ATG GAT GGA TGG AGA AGG ATG CCT CGC TGG GGA CTG CTG CTG CTG CTC TGG GGC TCC TGT  60
    Met Asp Gly Trp Arg Arg Met Pro Arg Trp Gly Leu Leu Leu Leu Leu Trp Gly Ser Cys
    1              {-60}        10                    {-50}              20

 61 ACC TTT GGT CTC CCG ACA GAC ACC ACC ACC TTT AAA CGG ATC TTC CTC AAG AGA ATG CCC 120
    Thr Phe Gly Leu Pro Thr Asp Thr Thr Thr Phe Lys Arg Ile Phe Leu Lys Arg Met Pro
                       {-40}        30                    {-30}              40

121 TCA ATC CGA GAA AGC CTG AAG GAA CGA GGT GTG GAC ATG GCC AGG CTT GGT CCC GAG TGG 180
    Ser Ile Arg Glu Ser Leu Lys Glu Arg Gly Val Asp Met Ala Arg Leu Gly Pro Glu Trp
                       {-20}        50                    {-10}              60

181 AGC CAA CCC ATG AAG AGG CTG ACA CTT GGC AAC ACC ACC TCC TCC GTG ATC CTC ACC AAC 240
    Ser Gln Pro Met Lys Arg Leu Thr Leu Gly Asn Thr Thr Ser Ser Val Ile Leu Thr Asn
                       {-1}{1}      70                    {10}              80

241 TAC ATG GAC ACC CAG TAC TAT GGC GAG ATT GGC ATC GGC ACC CCA CCC CAG ACC TTC AAA 300
    Tyr Met Asp Thr Gln Tyr Tyr Gly Glu Ile Gly Ile Gly Thr Pro Pro Gln Thr Phe Lys
                       {20}         90                    {30}              100

301 GTC GTC TTT GAC ACT GGT TCG TCC AAT GTT TGG GTG CCC TCC TCC AAG TGC AGC CGT CTC 360
    Val Val Phe Asp Thr Gly Ser Ser Asn Val Trp Val Pro Ser Ser Lys Cys Ser Arg Leu
                       {40}         110                   {50}              120

361 TAC ACT GCC TGT GTG TAT CAC AAG CTC TTC GAT GCT TCG GAT TCC TCC AGC TAC AAG CAC 420
    Tyr Thr Ala Cys Val Tyr His Lys Leu Phe Asp Ala Ser Asp Ser Ser Ser Tyr Lys His
                       {60}         130                   {70}              140

421 AAT GGA ACA GAA CTC ACC CTC CGC TAT TCA ACA GGG ACA GTC AGT GGC TTT CTC AGC CAG 480
    Asn Gly Thr Glu Leu Thr Leu Arg Tyr Ser Thr Gly Thr Val Ser Gly Phe Leu Ser Gln
                       {80}         150                   {90}              160

481 GAC ATC ATC ACC GTG GGT GGA ATC ACG GTG ACA CAG ATG TTT GGA GAG GTC ACG GAG ATG 540
    Asp Ile Ile Thr Val Gly Gly Ile Thr Val Thr Gln Met Phe Gly Glu Val Thr Glu Met
                       {100}        170                   {110}             180

541 CCC GCC TTA CCC TTC ATG CTG GCC GAG TTT GAT GGG GTT GTG GGC ATG GGC TTC ATT GAA 600
    Pro Ala Leu Pro Phe Met Leu Ala Glu Phe Asp Gly Val Val Gly Met Gly Phe Ile Glu
                       {120}        190                   {130}             200

601 CAG GCC ATT GGC AGG GTC ACC CCT ATC TTC GAC AAC ATC ATC TCC CAA GGG GTG CTA AAA 660
    Gln Ala Ile Gly Arg Val Thr Pro Ile Phe Asp Asn Ile Ile Ser Gln Gly Val Leu Lys
                       {140}        210                   {150}             220

661 GAG GAC GTC TTC TCT TTC TAC TAC AAC AGA GAT TCC GAG AAT TCC CAA TCG CTG GGA GGA 720
    Glu Asp Val Phe Ser Phe Tyr Tyr Asn Arg Asp Ser Glu Asn Ser Gln Ser Leu Gly Gly
                       {160}        230                   {170}             240

721 CAG ATT GTG CTG GGA GGC AGC GAC CCC CAG CAT TAC GAA GGG AAT TTC CAC TAT ATC AAC 780
    Gln Ile Val Leu Gly Gly Ser Asp Pro Gln His Tyr Glu Gly Asn Phe His Tyr Ile Asn
                       {180}        250                   {190}             260

781 CTC ATC AAG ACT GGT GTC TGG CAG ATT CAA ATG AAG GGG GTG TCT GTG GGG TCA TCC ACC 840
    Leu Ile Lys Thr Gly Val Trp Gln Ile Gln Met Lys Gly Val Ser Val Gly Ser Ser Thr
                       {200}        270                   {210}             280

841 TTG CTC TGT GAA GAC GGC TGC CTG GCA TTG GTA GAC ACC GGT GCA TCC TAC ATC TCA GGT 900
    Leu Leu Cys Glu Asp Gly Cys Leu Ala Leu Val Asp Thr Gly Ala Ser Tyr Ile Ser Gly
                       {220}        290                   {230}             300

901 TCT ACC AGC TCC ATA GAG AAG CTC ATG GAG GCC TTG GGA GCC AAG AAG AGG CTG TTT GAT 960
    Ser Thr Ser Ser Ile Glu Lys Leu Met Glu Ala Leu Gly Ala Lys Lys Arg Leu Phe Asp
                       {240}        310                   {250}             320

961 TAT GTC GTG AAG TGT AAC GAG GGC CCT ACA CTC CCC GAC ATC TCT TTC CAC CTG GGA GGC 1020
    Tyr Val Val Lys Cys Asn Glu Gly Pro Thr Leu Pro Asp Ile Ser Phe His Leu Gly Gly
                       {260}        330                   {270}             340

1021 AAA GAA TAC ACG CTC ACC AGC GCG GAC TAT GTA TTT CAG GAA TCC TAC AGT AGT AAA AAG 1080
    Lys Glu Tyr Thr Leu Thr Ser Ala Asp Tyr Val Phe Gln Glu Ser Tyr Ser Ser Lys Lys
                       {280}        350                   {290}             360

1081 CTG TGC ACA CTG GCC ATC CAC GCC ATG GAT ATC CCG CCA CCC ACT GGA CCC ACC TGG GCC 1140
    Leu Cys Thr Leu Ala Ile His Ala Met Asp Ile Pro Pro Pro Thr Gly Pro Thr Trp Ala
                       {300}        370                   {310}             380

1141 CTG GGG GCC ACC TTC ATC CGA AAG TTC TAC ACA GAG TTT GAT CGG CGT AAC AAC CGC ATT 1200
    Leu Gly Ala Thr Phe Ile Arg Lys Phe Tyr Thr Glu Phe Asp Arg Arg Asn Asn Arg Ile
                       {320}        390                   {330}             400

1201 GGC TTC GCG TTG GCC CGC TGAGGCCCTCTGCCACCCAGGCAGGCCCTGCCTTCAGCCCTGGCCCAGAGCTGGA 1273
    Gly Phe Ala Leu Ala Arg
                       {340}406

1274 ACACTCTCTGAGATGCCCCTCTGCCTGGGCTTATGCCCTCAGATGGAGACATTGGATGTGGAGCTCCTGCTGGATGCGT 1352

1353 GCCCTGACCCCTGCACCAGCCCTTCCCTGCTTTGAGGACAAAGAGAATAAAGACTTCATGTTCAC
```

[from Imai et al., Proc. Nat. Acad. Sci. USA 80:7405-7409(1983)]

# FIG. 1

FIG. 2

FIG. 3A

FIG. 3B

# FIG. 4

1 2 3 4 5 6

200 –

116 –
92 –

66 –

43 –

31 –

# FIG. 5

FIG. 6